# EUROPEAN PATENT SPECIFICATION

(11) **EP 1 618 913 B1**
(45) Date of publication and mention of the grant of the patent: **23.11.2011**
(21) Application number: 05254554.8
(22) Date of filing: 21.07.2005
(51) Int. Cl.: A61M 21/02

(54) **Device for insomnia assessment and automated sleep behaviour modification**
Vorrichtung zur Beurteilung von Schlaflosigkeit und zur automatisierten Modifizierung des Schlafverhaltens
Dispositif pour l'évaluation de l'insomnie et pour la modification automatisée du comportement de sommeil

(30) Priority: 23.07.2004 US 897035
(43) Date of publication of application: 25.01.2006
(73) Proprietor: PICS, Inc., Reston, VA 22091 (US)
(72) Inventor: Behar, Albert, Reston Virginia 20191 (US); Riley, William T., Ashburn Virginia 20147 (US)
(74) Representative: Kenrick, Mark Lloyd

(56) References cited:
- EP-A- 1 342 489
- WO-A-2004/078132
- DE-A1- 2 314 014
- US-A- 5 259 390
- US-B1- 6 392 962

## Description

### BACKGROUND OF THE INVENTION

The present invention relates to an insomnia assessment and treatment device (IATD) that adapts behavioral principles to assist patients suffering from insomnia.

Insomnia affects approximately 30 million people in the U.S. Few patients suffering from insomnia seek medical treatment from a health professional. Those who consult with a physician often receive treatments, such as drug medications, that have minimal long-term efficacy and sometimes exacerbate insomnia. There is a need for a system to treat insomnia that has long-term efficacy and may be used by patients regardless of whether they seek medical treatment from a health professional.

Conventional insomnia therapy typically involves a sleep assessment and an insomnia treatment. Sleep assessments determine the sleep patterns and sleep periods of a patient. Using a sleep assessment, a health professional typically prescribes an insomnia treatment that may include drugs, sleep aid devices, and/or behavior therapy.

Sleep assessment types include polysomnography and actigraphy. During polysomnography, EEG (brain waves), EMG (muscle movement as in restless leg syndrome), respiratory functions (for sleep apnea) and ocular movement (rapid eye movement or REM) of a patient are measured and analyzed by a computer and interpreted by trained sleep technicians or health professionals. Actigraphy is another common sleep assessment treatment that measures the asleep and awake states of a patient. Actigraph accelerometers, usually worn on the wrist of the patient, monitor the amount of physical activity of the patient. Based on the data collected from the accelerometers, the sleep periods of the patient may be measured by differentiating between the level of movement usually associated with wake states and sleep states. Actigraphy may be combined with other physiological monitoring, e.g., body temperature and galvanic skin response, to improve the accuracy of sleep monitoring.

Another sleep assessment technique is the use of sleep tracking devices, such as a "dead man's switch", to detect when a patient falls asleep. These tracking devices include a switch-activated clock started by the release of a dead-man's switch which the patient grasps when they go to bed and releases when they fall asleep. The release of the switch indicates that the patient is asleep and starts a timer in the sleep tracking device. Upon awakening, the patient records the elapsed time that he is asleep. The elapsed time may be displayed by the sleep tracking device. Exemplary sleep tracking devices using dead man's switches are disclosed in U.S. Patent Nos. 6,392,962 and 6,078,549.

Active sampling is another sleep assessment technique. Sampling involves periodically prompting a patient to respond to determine if the patient is asleep. An exemplary active sleep sampling device was developed by Lichstein and colleagues (Kelley & Lichstein, 1980) as the Sleep Assessment Device (SAD) which produced a short, low volume tone every 10 minutes throughout the night which the subject verbally acknowledges by stating "I'm awake" into a tape player. A health professional would later listen to the tape and manually record the response of the patient to each prompt. When the patient did not respond to the prompt, it was presumed that he/she was asleep. This sampling procedure was not found to be disruptive to the sleep of the insomnia patients. Sleep sampling has been found to be reliable for assessing sleep patterns.

The treatments for insomnia are pharmacologic and behavioral. Medical treatments include homeopathic remedies (e.g. melatonin), over the counter (OTC) non-prescription medications (diphenhydramine, Unisom) and prescribed medications (Ambien). A range of peripheral products have been developed to provide an environment conducive to sleep. They include: sleep aids such as pillows that cool the patient, white noise generators and relaxing music players. In addition, electronic sleep aid products include an acupuncture device called "Alpha-Stim SCS" which emits small electrical currents to the ear lobe and induces relaxation and sleep, and a magnetic field generator which supposedly induces sleep (low energy emission therapy).

Behavioral treatments for insomnia include training sleep hygiene (e.g. wind down time to relax before going to bed), cognitive restructuring (e.g. break the cycle of trying so hard to sleep that you become tense and unable to sleep), relaxation training, stimulus control (associate getting into bed with falling asleep) and sleep restriction (cut back on time in bed and gradually shape sleep behavior). These behavioral treatments have typically been delivered by health professionals with expertise in insomnia. Books such as Peter Hauri's "No More Sleepless Nights" and Charles Morin's "Relief From Insomnia: Getting the Sleep of Your Dreams" provide these behavioral sleep strategies in a self-help form.

Many who have researched self-help insomnia treatments suggest that some of the most efficacious components of the treatment, i.e., stimulus control and sleep restriction, are difficult to teach to patients and to assure compliance by the patients. Accordingly, there is a long felt need for sleep assessments techniques and insomnia treatments, especially those that teach and monitor compliance of stimulus control, sleep restriction and other insomnia treatment components.

### BRIEF DESCRIPTION OF THE INVENTION

In one embodiment, the invention provides a device for automated sleep behavior modification, the device comprising: a memory arranged to store sleep behavior data; a processor arranged to generate a sleep behavior regimen based on the stored sleep behavior data and a behavior modification algorithm, wherein the algorithm generates at least one sleep behavior prompt; an output arranged to provide the at least one sleep behavior prompt for a user to perform a predetermined sleep inducement behavior at a time determined by the sleep behavior regimen, wherein said sleep behavior prompt comprises a prompt for getting in bed; an input arranged to collect data indicative of whether the user is awake or asleep; the memory being further arranged to store the data indicative of whether the user is awake or asleep, after the user has been prompted to get in bed; the processor being further arranged to use the stored data to determine a sleep efficiency based on a ratio of a period that the user is asleep and a period that the user is in bed and generate a modified sleep behavior regimen based on the sleep efficiency; and the output being further arranged to provide the prompt for getting in bed at a time determined by the modified sleep behavior regimen.

The predetermined sleep inducement behavior may be prompting a prescribed bedtime and wake-up time.

Prompting the sleep inducement behavior may comprise prompting the user to get out of bed if the user does not fall asleep within a predetermined time after getting into bed.

Data regarding the baseline sleep pattern data may be collected by monitoring the user during at least one sleep period.

Data regarding the baseline sleep pattern data may be collected by tracking a response of the user to a series of sleep behavior prompts periodically issued throughout a sleep period.

Data regarding the baseline sleep pattern data may be collected by determining when the user releases a dead man's switch.

Data regarding the baseline sleep pattern data may be collected by actively sampling responses of a user to a series of sleep behavior prompts given while the user is in bed.

Data regarding the baseline sleep pattern data may comprise baseline sleep data manually entered into the system.

The regimen may be maintained for a period without further modification after a desired sleep behavior has been achieved.

A sleep pattern of a user may be assessed and data regarding the sleep pattern may be stored. A regimen to improve sleep may be generated. The user may be prompted to perform the sleep inducement behaviors as prescribed by the regimen, and the sleep pattern of the user may be tracked and data stored regarding the tracked sleep pattern. The sleep inducement regimen based on the tracked sleep pattern may be modified and the user may be prompted to perform a predetermined sleep inducement behavior prescribed by the modified regimen.

The predetermined sleep behavior may be the time the patient gets into bed.

Assessing the sleep pattern may comprise monitoring the user's sleep for a period of time.

Assessing the sleep pattern may comprise periodically prompting the user to respond if awake and tracking the response of the user.

Assessing the sleep pattern may comprise monitoring when the user releases a dead man's switch.

Assessing the sleep pattern may comprise actively sampling responses of a user to a series of prompts given while the user is in bed.

The sleep pattern assessment may comprise baseline sleep data manually entered into the system.

The modified regimen may be maintained for a period without further modification after a desired sleep behavior has been achieved.

The stored sleep behavior data may be collected by monitoring a user's sleep for a period of time.

The stored sleep behavior data may be collected by prompting the user to respond if awake to a prompt; or responds involuntarily or physiologically to a prompt.

The stored sleep behavior data may be collected by monitoring when the user releases a dead man's switch.

The stored sleep behavior data may be collected by actively sampling responses to a user to a series of prompts given while the patient is in bed.

The stored sleep behavior may comprise baseline sleep data manually entered into the system.

The regimen may be maintained for a period without further modification after desired sleep behavior has been achieved.

A sleep behavior pattern of a user operating a computer controlled treatment device may be assessed using a method comprising: the device actively sampling the user to periodically determine whether the user is awake or asleep; the device automatically collecting data indicative of whether the user is awake or asleep during the active sampling, and determining a sleep behavior pattern of the user based on the collected data.

The step of active sampling may comprise periodically prompting the user to respond if awake, and the step of automatic collecting may comprise the device recording as the data the responses and lack of responses to the prompts.

The step of active sampling may comprise periodically issuing a prompt to the user while the user is in bed and the step of automatically collecting data may be recording whether the user responds to each prompt.

The step of determining the sleep behavior the collected data may be used to assess total sleep time of the user while in bed.

The collected data may be used to assess sleep onset latency.

The collected data may be used to assess sleep efficiency of the user.

The sleep behavior pattern may be manually inputted into the device.

The user prompt may be a speaker issuing an audible signal.

The user input may be a key actuated by the user.

The sleep behavior may be a prescribed bedtime.

The device may further comprise a second input to receive a response by the user to a second prompt, wherein the second prompt is periodically generated by the processor to actively sample the sleep pattern of the user.

### BRIEF DESCRIPTION OF THE DRAWINGS

FIGURE 1 is a diagram of a patient in a bed using an insomnia assessment and treatment device (IATD) device.
FIGURE 2 is a flow chart of steps to be performed to assess and treat insomnia.
FIGURE 3 a front view of the IATD showing the user interface of the device.
FIGURE 4 is a schematic block diagram of the electronic components of the IATD.

### DETAILED DESCRIPTION OF THE INVENTION

An Insomnia Assessment and Treatment Device (IATD) has been developed. In various embodiments, the IATD may be used to provide computerized or automated sleep assessments, and sleep behavioral treatment, such as nonpharmacologic treatment or psychosocial treatment of insomnia. The IATD is configured to assist a user to follow regimens for insomnia assessment and treatment that have been automated and embodied in the IATD device. For example, the IATD may automatically and continually assess the sleep behavior of a patient, produce prompts to train the user to get out of bed if not asleep within a specified period (which is stimulus control), and signal the user when to go to bed or wake up (which is sleep restriction). The program(s) implemented by the IATD is based on established and scientifically supported sleep assessments and behavioral strategies using stimulus control and sleep restriction. The user may use the IATD as a self-help insomnia program or under the guidance of a physician or other heath care professional.

Insomnia is typically defined as one or more months of complaints of difficulty initiating or maintaining sleep or non-restorative sleep which causes distress or impairs normal functioning of a patient. The diagnosis of insomnia is subjective. Insomnia does not easily fit into a simple definition. Primary insomnia (also referred to as psychophysiological insomnia) is insomnia that is not due to another condition such as sleep apnea, pain or depression. The IATD disclosed herein is most helpful in treating primary insomnia, but may also be applicable in treating other types of insomnia (especially when combined with other treatments for apnea, pain, depression or other contributing condition).

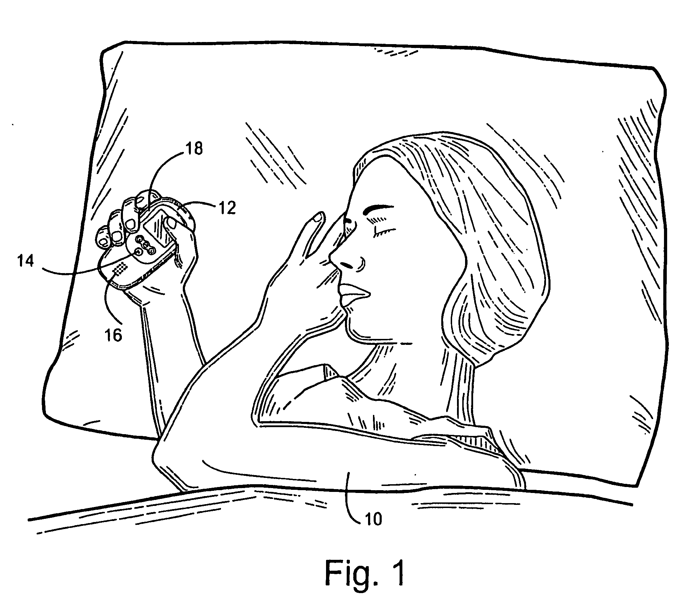

FIGURE 1 is a diagram of a user 10 in a bed using an IATD 12. The user lies in her bed in a normal fashion and prepares to go to sleep. Upon entering her bed, the user presses a "bed" key 14 on the IATD. The IATD records the time when she enters her bed. The user, while laying in bed, holds the IATD instrument 12 in her hand. A strap may secure the IATD instrument to the hand.

The IATD 12 may use an active sampling technique to track sleep behavior. Active sampling may be the IATD periodically, e.g., every ten minutes, prompting the user to determine if she is awake or asleep. The IATD 12 may audibly prompt the user by emitting a sound through a speaker 16. When prompted, the user activates the awake bar 18. If the user does not activate the bar 18 within a predetermined period, e.g. ten seconds, the IATD 12 assumes that the user is asleep. The IATD records the time of the prompt (or when the bar is pressed) and whether the awake bar is depressed as an indication of the user being awake or asleep.

The audible prompt may be a low tone, low volume pulse sound emitted periodically, e.g., every ten minutes. This pulse sound is not disruptive to sleep but can be heard by the patient if awake. As an alternative to or in addition to the audible prompt, the IATD device may emit a light prompt, may vibrate or otherwise prompt the user to respond if awake. If the user is asleep, the prompt is sufficiently quiet and subtle so as not to disturb the sleep state.

The response by the user to the IATD may be depressing the awake bar 18, or by a verbal response or by general movement (such as shaking an actigraph in response to a prompt). Moreover, the user's response to the IATD may be voluntary or involuntary depending on sleep sensing probe or prompt. Based on the user's response or lack of a response, the IATD automatically determines awake or asleep states.

Regardless of whether the user depresses the awake bar, the IATD may continue to sample sleep by emitting the audible prompt periodically. By sampling, the IATD determines approximately the periods of the user's awake state and/or asleep state while in bed. Active sampling enables the IATD to collect data regarding the period that a user lies awake in bed until she falls asleep and any subsequent awake periods while in bed. The device 12 also collects data on the period(s) that the patient is asleep. By active sampling, the IATD collects data regarding the patient's sleep behavior. These data are collected so that the IATD can assess sleep patterns.

The IATD stores the timing of each prompt and whether the user responded to the prompt. The stored time and response data are later analyzed by the IATD to assess the sleep pattern of a user. For example, the data are used to determine approximately the period between when a patient enters the bed and falls asleep, the period(s) of sleep, the number and length of awakenings during the night, and when the user gets out of bed.

The user indicates to the IATD when she is in or out of bed either via key 14, by docking the IATD into cradle, and/or pressing or releasing a pressure pad. The IATD provides automatic and immediate computation each morning of relevant sleep variables such as total time in bed, total time asleep, sleep onset latency (time from initial in bed to asleep), and sleep efficiency (time asleep/time in bed) with feedback to the user about these variables each morning.

The core functions of the IATD 12 may include one or more of the following functions:
(i) Measurement of asleep vs. awake periods. While the user is in bed, active sleep sampling is employed to periodically prompt the patient to respond by pressing the awake bar 18 if she is still awake. The prompt may be a regularly scheduled low tone, low volume beep (sleep tone) emitted by the device. Absence of a response, e.g., the bar 18 is not pressed, is assumed to indicate that the patient is asleep. By periodically repeating the sleep tone, e.g., every 10 minutes, the device 12 may track the sleep period(s) of the patient and determine how often the patient awakens during the night. As an alternative to active sampling, sleep periods may measured by a "dead man's switch" in which a button is held by the user when they go to bed and released when they fall asleep.
(ii) Sleep restriction. The IATD sets a regular wake time or allows a user to set a regular wake time. The regular wake time may be a certain time of day, e.g., 8:00 am, or after a regular sleep period, e.g., eight hours of sleep. The IATD emits alarms, e.g., an audible alarm 16, sufficient to cause the user to wake up at a prescribed awake time. Awakening the patient at a prescribed wake time avoids excessive sleep, which can contribute to difficulty falling asleep the next night. The IATD may also emit a prompt at a time scheduled to go to bed. This bedtime prompt encourages the user to restrict the amount of time in bed. The bedtime prompt may be automatically determined by the IATD based on the assessed time asleep from a prior night(s).
(iii) Stimulus control. The IATD trains a user to fall asleep in response to getting in bed. The IATD prompts the patient to get out of bed if she is not asleep within 20 minutes, for example. Prompting the user to get out of bed provides sleep stimulus control. The stimulus is the act of getting into bed. The desired response to this stimulus is that the patient falls asleep shortly after getting into bed. If she does not quickly fall asleep, the stimulus of getting into bed is being associated with not sleeping so she is prompted to get out of bed to break this association. The user may be instructed not to return to bed until after a period of time, e.g., 15 minutes, has passed or the patient is sleepy. When the user returns to bed, the stimulus period is restarted and the IATD 12 resumes active sampling to check whether she falls asleep within 20 minutes. This process of getting into bed, attempting to fall asleep in 20 minutes, getting out of bed after 20 minutes if not asleep, and repeating the process is sleep stimulus control training. Training modifies the behavior of the user such that she will respond to the stimulus of getting into bed by falling asleep.
(iv) The IATD adjusts the bed time based on the user's sleep efficiency (the ratio of time asleep/time in bed) and gradually shapes the sleep behavior of the patient to a regular and normal sleep pattern. The IATD shapes the sleep behavior by scheduling and prompting bedtime and gradually making this bedtime earlier or later based on the patient's sleep efficiency. A high sleep efficiency results when the patient is asleep during the vast majority of time that she is in bed. It is desirable that the efficiency be high such that the vast majority of time in bed is asleep time.

The IATD provides computerized and automated delivery of sleep restriction for insomnia by use of active sampling (signaling to check if awake) of sleep to compute (automated via a computer device 12) daily sleep variables that can be identified and, the IATD can provide immediate feedback to patient each morning. The IATD uses active or passive behavioral sleep sampling to automatically produce and adjust the behavioral insomnia treatments programmed into the IATD, e.g., a prescribed bedtime each night. The IATD also provides computerized and automated delivery of stimulus control for insomnia. Further, the IATD provides the ability to provide continuous collection of data for sleep assessments which can be used to track changes in the sleep behavior of a patient that, for example, result from the sleep stimulus and sleep restriction regimens generated by the IATD for the user. Further, the IATD can adjust the sleep stimulus and sleep restriction regimens based on new data collected from the ongoing sleep data collected by the sleep assessment operation of the IATD.

The IATD shown in Figure 1 is a dedicated handheld electronic device. The IATD may also be implemented, for example, on a desktop computer, a personal digital assistant (PDA), a smart phone or an Internet web program in which a patient self-reports information from the night before and the web program sets up the next day's sleep schedule for the patient based on the entered data and sleep assessment algorithms.

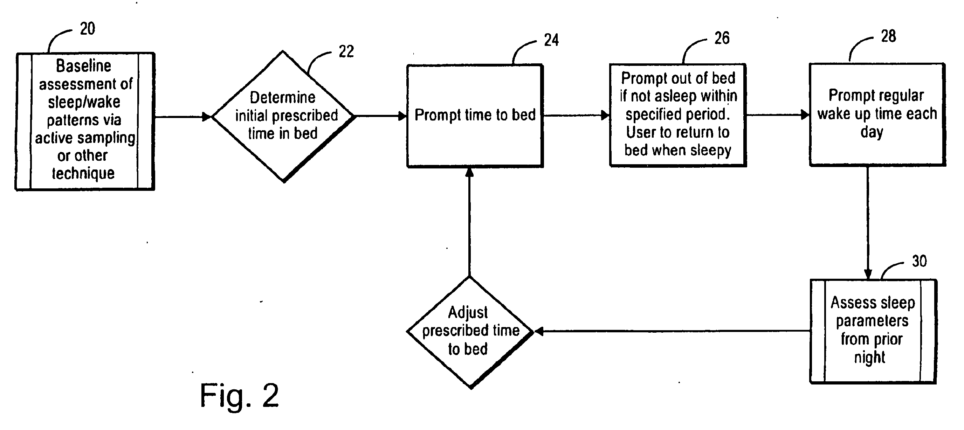

FIGURE 2 is a flow chart of a method to assess the sleep behavior of a user and to automatically prescribe behavioral treatments, to alleviate insomnia and restore normal sleep. The sleep and awake states, as well as associated sleep variables, are determined in an automated fashion by the IATD. The IATD automatically assesses asleep and awake states by recording responses of the user to a periodic prompt and storing that data in ROM.

Based on initial baseline sleep data 20, the IATD determines the following to design a tailored insomnia treatment approach that may assess or set one or more of the following: a) total time in bed, b) bedtime, c) sleep onset latency (which is the approximate period from when the user enters her bed and falls asleep), d) morning awake times, e) total sleep time (which may be approximated based on the response by the user to the periodic prompts), and f) sleep efficiency (which is the ratio of the approximate period that the user is asleep to the period that the user is in bed).

The IATD encourages the user to set a morning awake time with minimal variability (consistent wake up time) and prompts the user out of bed each morning (alarm clock function) at the awake time, in step 28. The IATD sets the time to bed each night based on prior sleep data and may display this time to the user on the IATD display screen 19. Initially, bedtime is set based on morning awake time and mean total sleep time. For example, if the user sleeps an average of six hours and the wake up time is 7:00 am, the IATD sets the bedtime for the next night for 1:00 am.

The IATD prompts the user to go to bed (auditory and visual display) at the time it determines based on the wake time and sleep assessment data, in step 24. If the user is not asleep within a specified time (e.g., 20-30 minutes), the IATD prompts her to get out of bed for a period of time before retrying to go to sleep (auditory and visual display) in step 26.

The IATD continues to assess the sleep time each night using methods specified above, e.g., active sampling, and adjusts the time to bed each night based on sleep efficiency from prior nights in step 30. High sleep efficiencies (e.g., greater than 85-90%) result in an earlier prescribed bedtime and low sleep efficiencies (<80%) result in a later prescribed bedtime. Accordingly, the bedtime may be automatically determined by the IATD based solely or in part on the sleep efficiency of the prior night(s) sleep.

The user indicates to the IATD when sleep is satisfactory. At that time, the user can switch the IATD to a "maintenance mode" which will continue to assess sleep and prompt her to get in and out of bed at prescribed times. In maintenance mode, the IATD does not make further adjustments to the sleep schedule (unless the sleep behavior worsens).

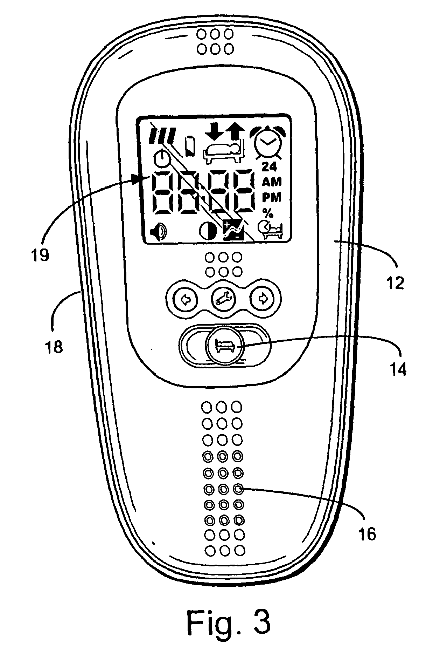

FIGURE 3 is a front view of a hand held IATD 12. The device includes a display, user input buttons and an audio speaker 16. Housed within the device are a computer processor and other electronics for operating the device. The IATD 12 fits easily in the palm of a hand of a user. The device may have minimal buttons and other user interface features to simplify its operation. For example, the primary user inputs may be a one large key bar 18 for responding to a prompt of the machine as to whether the user is awake or not, and a smaller "In Bed" button 14 for use when the user has entered the bed or gotten out of bed. The other buttons may be relatively small so as to avoid any tendency of the user hitting the wrong button when in a half asleep state.

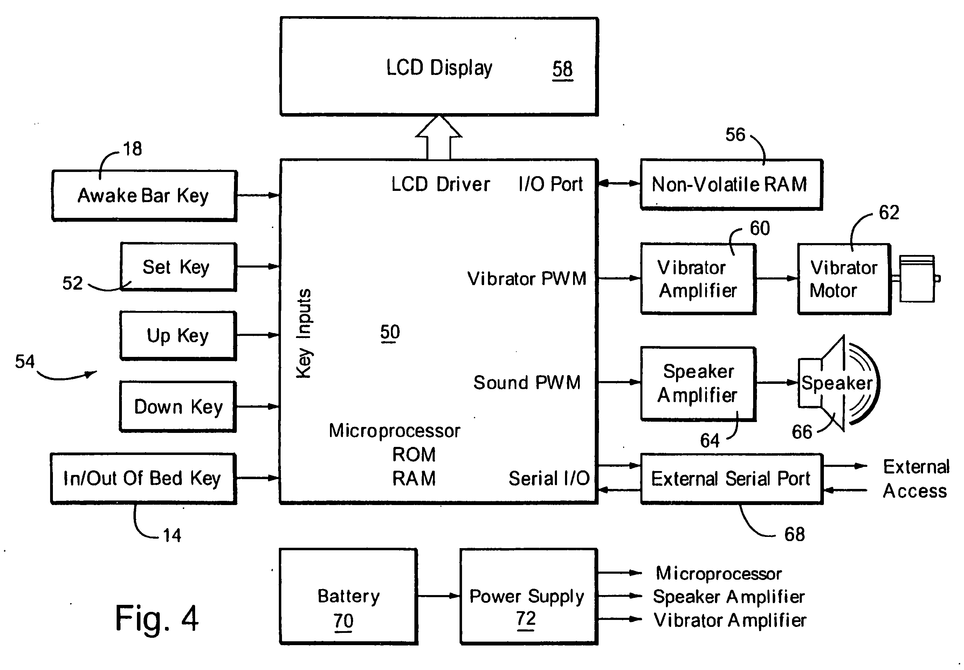

FIGURE 4 is an electronic schematic diagram of the computer processor and other electronics within the IATD 12. The processor 50 may be a conventional microprocessor having internal read only memory (ROM) and random access memory (RAM). The processor includes data and command inputs from the various keys on the device, such as the awake bar 18, in/out of bed key 14, a control set key 52, and up and down keys. These operator inputs are used by the user to enter data and user selections into the microprocessor.

The microprocessor executes a program stored in its ROM and/or the non-volatile RAM 56. The program may implement the method for sleep assessment and insomnia treatment, such as shown in Figure 2.

The processor 50 drives a liquid crystal display 58 which provides information to the patient regarding the operation of the device, the recorded sleep data and the sleep regimen. In addition, the processor may, when appropriate, activate a vibrational amplifier 60 and vibrator motor 62 to prompt the user to perform a certain act, such as to get out of bed or press the key bar 18. Similarly, the processor may output a sound to a speaker amplifier 64 and speaker 66 to audibly prompt the patient. Further, the processor may have an external serial port 68 to allow an external device, such as a computer, to interrogate data on the device and otherwise exchange data and control information with the device.

To power the device, a standard battery 70 powers a power supply 72 in the device which provides power to the microprocessor, speaker amplifier and vibrator amplifier. Further, the devices may include a non-volatile memory which permanently stores the executable programs of the device and does not allow erasure of the programs when the battery expires or is replaced.

The processor 50 and the algorithm programs and sleep data stored in its associated memory ROM, RAM and non-volatile RAM 56 provide the logic to perform sleep assessments, automatically generate a sleep modification behavior regimen and analyze data regarding sleep behaviors to determine if and when the regimen should be modified. This logic may in other embodiments be implemented in other devices, such as personal computers; personal digital assistants (PDAs); smart cell or wired telephones (where "smart" refers to processors in the telephone or another device in communication with the telephone that enable users or manufacturers to embed functions such as an IATD program), and smart televisions, digital alarm clocks and other consumer electronic products. Further, the IATD logic functions may be on a central computer system that a user interacts with over, for example, a telephone connection or Internet website communication link.

By way of example, the logic means on a remote computer system may be accessible to a user via an Internet website that collects user information such as patient identification, and user baseline sleep behavior. The information collected from the user may be manually entered to the website or automatically entered by a monitoring device, such as a hand-held device, user movement sensor or other device that tracks sleep behavior. The monitoring device transmits, via a wired or wireless link, sleep behavior information to a data entry device, such as a personal computer or PDA, that enters data to the website of the IATD logic means. The IATD logic means applies the sleep behavioral algorithms, such as shown in Figure 2, to automatically generate a sleep inducement regimen.

The sleep inducement regimen is initially generated using baseline sleep behavior data that are manually entered by the user and/or based on sleep behavior data collected by a monitoring device. For example, the user may enter in information regarding his or her age, sex, desired wake up time and times in bed and asleep for each of the seven days of the week or a monitoring device collects sleep behavior data from one or more nights sleep (before a sleep inducement behavior regimen is generated). The regimen may be modified based on, for example, sleep data collected during one or more prior nights of sleep. The regimen prescribes sleep inducement behaviors, such as what time to go to bed and how long to lay awake in bed before getting out of bed. The website outputs prompts for the prescribed sleep inducement behavior, such as a "go to bed" prompt and a "get out of bed" prompt. The prompts are transmitted from the website via the Internet to the patient's personal computer, PDA, telephone or the like which issues the prompt to the patient; or tailored instructions for self-prompting of the next night's sleep could be provided to the user.

While the invention has been described in connection with what is presently considered to be the most practical and preferred embodiment, it is to be understood that the invention is not to be limited to the disclosed embodiment, but on the contrary, is limited only by the scope of the appended claims.

## Claims

1. A device (12) for automated sleep behavior modification, the device comprising:
a memory (56) arranged to store sleep behavior data (20);
a processor (50) arranged to generate a sleep behavior regimen based on the stored sleep behavior data (20) and a behavior modification algorithm (22), wherein the algorithm generates at least one sleep behavior prompt;
an output (60, 66, 58) arranged to provide the at least one sleep behavior prompt for a user to perform a predetermined sleep inducement behavior at a time determined by the sleep behavior regimen, wherein said sleep behavior prompt comprises a prompt (24) for getting in bed;
an input (14,18) arranged to collect data (30) indicative of whether the user is awake or asleep;
the memory (56) being further arranged to store the data (30) indicative of whether the user is awake or asleep, after the user has been prompted to get in bed;
the processor (50) being further arranged to use the stored data (30) to determine a sleep efficiency based on a ratio of a period that the user is asleep and a period that the user is in bed and generate a modified sleep behavior regimen based on the sleep efficiency, and
the output being further arranged to provide the prompt (24) for getting in bed at a time determined by the modified sleep behavior regimen.

2. A device as claimed in claim 1 wherein said at least one sleep behavior prompt further comprises at least one of a prompt (26, 28) for getting out of bed, and waking-up.

3. A device as claimed in any one of the above claims, wherein the predetermined sleep inducement behavior comprises prompting (26) the user to get out of bed if the user does not fall asleep within a predetermined time after getting into bed.

4. A device as claimed In any one of the above claims, wherein said input comprises a dead man's switch (18), said data indicative of whether the user is awake or asleep being collected by determining when the user releases the dead man's switch after the prompt for getting in bed.

5. A device as claimed in any of the above claims, wherein the input (14, 18) is arranged to actively sample responses of the user to a series of sleep behavior prompts for collecting said data indicative of whether the user is awake or asleep.

6. A device as claimed in any one of the above claims, wherein said input (14, 18) is arranged for manually entering sleep behavior data.

7. A device as claimed in any one of the above claims, wherein the device (12) **is** arranged to maintain the sleep behavior regimen for a period without further modification, after data indicative of a predetermined desired sleep behavior has been received.

8. A carrier medium (12) carrying computer readable program code configured to cause a computer having a processor (50), a memory (56), an input (14, 18) and an output (60, 66, 58) to carry out of a method of:
storing (20, 56) sleep behavior data (20) in the memory (56);
generating, at the processor (50), a sleep behavior regimen based on the stored sleep behavior data (20) and a behavior modification algorithm (22), wherein the algorithm generates at least one sleep behavior prompt;
providing, from the output (60, 66, 58), a sleep behavior prompt for a user to perform a predetermined sleep inducement behavior at a time determined by the sleep behavior regimen, wherein said sleep behaviour prompt comprises a prompt (24) for getting in bed;
collecting, from the input (14, 18), data (30) indicative of whether the user is awake or asleep;
storing, in the memory (56), the data (30) indicative of whether the user is awake or asleep, after the user has been prompted to get in bed;
using the stored data (30) to determine, at the processor (50), a sleep efficiency based on a ratio of a period that the user is asleep and a period that the user is in bed and generating, at the processor (50), a modified sleep behavior regimen based on the sleep efficiency; and
providing, from the output (60, 66, 58), the prompt (24) for getting in bed at a time determined by the modified sleep behavior regimen.

## Patentansprüche

1. Einrichtung (12) zur automatischen Schlafverhaltensmodifikation, wobei die Einrichtung Folgendes umfasst:
einen Speicher (56), angeordnet zum Speichern von Schlafverhaltensdaten (20);
eine Prozessor (50), angeordnet zum Erzeugen eines Schlafverhaltensregimes auf der Basis der gespeicherten Schlafverhaltensdaten (20) und eines Verhaltensmodifikationsalgorithmus (22), worin der Algorithmus mindestens einen Schlafverhaltensprompt erzeugt;
einen Ausgang (60, 66, 58), angeordnet zum Bereitstellen des mindestens einen Schlafverhaltensprompts für einen Benutzer, um ein vorbestimmtes Schlafanreizverhalten zu einem Zeitpunkt auszuführen, der durch das Schlafverhaltensregime bestimmt wird, worin der Schlafverhaltensprompt einen Prompt (24) zum Zu-Bett-Gehen umfasst;
einen Eingang (14, 18), dazu angeordnet, Daten (30) zu sammeln, die anzeigen, ob der Benutzer wach oder schlafend ist;
wobei der Speicher (56) außerdem dazu geordnet ist, die Daten (30) zu speichern, die anzeigen, ob der Benutzer wach oder schlafend ist, nachdem der Benutzer einen Prompt zum Zu-Bett-Gehen empfangen hat,
wobei der Prozessor (50) außerdem dazu angeordnet ist, die gespeicherten Daten (30) dazu zu verwenden, eine Schlafeffizienz auf der Basis eines Verhältnisses einer Periode zu bestimmen, während der der Benutzer schläft, und einer Periode, während der der Benutzer im Bett liegt, und ein modifiziertes Schlafverhaltensregime auf der Basis der Schlafeffizienz zu bestimmen; und
der Ausgang außerdem dazu angeordnet ist, den Prompt (24) zum Zu-Bett-Gehen zu einem Zeitpunkt bereitzustellen, der durch das modifizierte Schlafverhaltensregime bestimmt wird.

2. Einrichtung nach Anspruch 1, worin der mindestens eine Schlafverhaltensprompt außerdem mindestens einen von Folgenden umfasst: einen Prompt (26, 28) zum Aufstehen und zum Aufwachen.

3. Einrichtung nach einem der obigen Ansprüche, worin das vorbestimmte Schlafanreizverhalten umfasst, dass der Benutzer einen Prompt (26) zum Aufstehen empfängt, falls der Benutzer nicht innerhalb einer vorbestimmten Zeit nach dem Zu-Bett-Gehen einschläft.

4. Einrichtung nach einem der obigen Ansprüche, worin der Eingang einen Totmannsschalter (18) umfasst, wobei die Daten, die anzeigen, ob der Benutzer wach oder schlafend ist, gesammelt werden, indem bestimmt wird, wann der Benutzer nach dem Prompt zum Zu-Bett-Gehen den Totmannsschalter löst.

5. Einrichtung nach einem der obigen Ansprüche, worin der Eingang (14,18) dazu angeordnet ist, Reaktionen des Benutzers auf eine Reihe von Schlafverhaltensprompts aktiv abzufragen, um die Daten zu sammeln, die anzeigen, ob der Benutzer wach oder schlafend ist.

6. Einrichtung nach einem der obigen Ansprüche, worin der Eingang (14,18) dazu angeordnet ist, Schlafverhaltensdaten manuell einzugeben.

7. Einrichtung nach einem der obigen Ansprüche, worin die Einrichtung (12) dazu angeordnet ist, das Schlafverhaltensregime für eine Periode ohne weitere Modifikation aufrechtzuerhalten, nachdem Daten empfangen wurden, die ein vorbestimmtes gewünschtes Schlafverhalten anzeigen.

8. Trägermedium (12), das computerlesbaren Programmcode trägt, konfiguriert zum Bewirken, dass ein Computer mit einem Prozessor (50), einem Speicher (56), einem Eingang (14, 18) und einem Ausgang (60, 66, 58) ein Verfahren von Folgenden ausführt:
Speichern (20, 56) von Schlafverhaltensdaten (20) im Speicher (56);
am Prozessor (50) ein Schlafverhaltensregime auf der Basis der gespeicherten Schlafverhaltensdaten (20) und einem Verhaltensmodifikationsalgorithmus (22) erzeugen, worin der Algorithmus mindestens einen Schlafverhaltensprompt erzeugt;
vom Ausgang (60, 66, 58) einen Schlafverhaltensprompt bereitstellen, damit ein Benutzer ein vorbestimmtes Schlafanreizverhalten zu einem Zeitpunkt ausführt, der durch das Schlafverhaltensregime bestimmt wird, worin der Schlafverhaltensprompt einen Prompt (24) zum Zu-Bett-Gehen umfasst;
vom Eingang (14, 18) Daten (30) sammeln, die anzeigen, ob der Benutzer wach oder schlafend ist;
im Speicher (56) die Daten (30) speichern, die anzeigen, ob der Benutzer wach oder schlafend ist, nachdem der Benutzer einen Prompt zum Zu-Bett-Gehen empfangen hat;
Verwenden der gespeicherten Daten (30), um am Prozessor (50) eine Schlafeffizienz zu bestimmen auf der Basis eines Verhältnisses einer Periode, während der der Benutzer schläft, und einer Periode, während der der Benutzer im Bett ist, und am Prozessor (50) ein modifiziertes Schlafverhaltensregime auf der Basis der Schlafeffizienz erzeugen; und
vom Ausgang (60, 66, 58) den Prompt (24) zum Zu-Bett-Gehen zu einem Zeitpunkt bereitstellen, der durch das modifizierte Schlafverhaltensregime bestimmt wird.

## Revendications

1. Dispositif (12) de modification automatisée du comportement au cours du sommeil, le dispositif comprenant :
une mémoire (56) agencée de manière à stocker des données de comportement au cours du sommeil (20) ;
un processeur (50) agencé de manière à générer un schéma de comportement au cours du sommeil sur la base des données de comportement au cours du sommeil stockées (20) et d'un algorithme de modification du comportement (22), dans lequel l'algorithme génère au moins une incitation de comportement au cours du sommeil ;
une sortie (60, 66, 58) agencée de manière à fournir ladite au moins une incitation de comportement au cours du sommeil permettant à un utilisateur d'adopter un comportement à l'endormissement prédéterminé à un instant déterminé par le schéma de comportement au cours du sommeil, ladite incitation de comportement au cours du sommeil comportant une incitation (24) à se mettre au lit ;
une entrée (14, 18) agencée de manière à recueillir des données (30) indiquant si l'utilisateur est éveillé ou endormi ;
la mémoire (56) étant en outre agencée de manière à stocker les données (30) indiquant si l'utilisateur est éveillé ou endormi, après que l'utilisateur a été incité à se mettre au lit ;
le processeur (50) étant en outre agencé de manière à utiliser les données stockées (30) en vue de déterminer une efficacité du sommeil sur la base d'un rapport entre une période au cours de laquelle l'utilisateur est endormi et une période au cours de laquelle l'utilisateur est au lit, et à générer un schéma de comportement au cours du sommeil modifié sur la base de l'efficacité du sommeil ; et
la sortie étant en outre agencée de manière à fournir l'incitation (24) à se mettre au lit à un instant déterminé par le schéma de comportement au cours du sommeil modifié.

2. Dispositif selon la revendication 1, dans lequel ladite au moins une incitation de comportement au cours du sommeil comprend en outre au moins une incitation (26, 28) à se réveiller et à sortir du lit.

3. Dispositif selon l'une quelconque des revendications précédentes, dans lequel le comportement à l'endormissement prédéterminé comprend l'étape consistant à inciter (26) l'utilisateur à sortir du lit si l'utilisateur ne s'endort pas au cours d'un temps prédéterminé après s'être mis au lit.

4. Dispositif selon l'une quelconque des revendications précédentes, dans lequel ladite entrée comporte un commutateur d'homme mort (18), lesdites données indiquant si l'utilisateur est éveillé ou endormi étant recueillies en déterminant le moment où l'utilisateur libère le commutateur d'homme mort à l'issue de l'incitation à se mettre au lit.

5. Dispositif selon l'une quelconque des revendications précédentes, dans lequel l'entrée (14, 18) est agencée de manière à échantillonner activement des réponses de l'utilisateur à une série d'incitations de comportement au cours du sommeil pour collecter lesdites données indiquant si l'utilisateur est éveillé ou endormi.

6. Dispositif selon l'une quelconque des revendications précédentes, dans lequel ladite entrée (14, 18) est agencée de manière à saisir manuellement des données de comportement au cours du sommeil.

7. Dispositif selon l'une quelconque des revendications précédentes, dans lequel le dispositif (12) est agencé de manière à conserver le schéma de comportement au cours du sommeil pendant une période de temps, sans réaliser de modifications supplémentaires, après que des données indiquant un comportement au cours du sommeil désiré prédéterminé ont été reçues.

8. Support de transport (12) transportant un code de programme lisible par un ordinateur, configuré de manière à amener un ordinateur présentant un processeur (50), une mémoire (56), une entrée (14, 18) et une sortie (60, 66, 58) à mettre en oeuvre un procédé comportant les étapes ci-dessous consistant à :
stocker (20, 56) des données de comportement au cours du sommeil (20) dans la mémoire (56) ;
générer, au niveau du processeur (50), un schéma de comportement au cours du sommeil sur la base des données de comportement au cours du sommeil stockées (20) et d'un algorithme de modification du comportement (22), dans lequel l'algorithme génère au moins une incitation de comportement au cours du sommeil ;
fournir, à partir de la sortie (60, 66, 58), une incitation de comportement au cours du sommeil permettant à un utilisateur d'adopter un comportement à l'endormissement prédéterminé à un instant déterminé par le schéma de comportement au cours du sommeil, dans laquelle ladite incitation de comportement au cours du sommeil comporte une incitation (24) à se mettre au lit :
collecter, à partir de l'entrée (14, 18), des données (30) indiquant si l'utilisateur est éveillé ou endormi ;
stocker, dans la mémoire (56), les données (30) indiquant si l'utilisateur est éveillé ou endormi, après que l'utilisateur a été incité à se mettre au lit ;
utiliser les données stockées (30) en vue de déterminer, au niveau du processeur (50), une efficacité du sommeil sur la base d'un rapport entre une période au cours de laquelle l'utilisateur est endormi et une période au cours de laquelle l'utilisateur est au lit, et générer, au niveau du processeur (50), un schéma de comportement au cours du sommeil modifié sur la base de l'efficacité du sommeil ; et
fournir, à partir de la sortie (60, 66, 58), l'incitation (24) à se mettre au lit à un instant déterminé par le schéma de comportement au cours du sommeil modifié.
